Europäisches Patentamt

**European Patent Office**

Office européen des brevets

(19)

(11) Publication number: **0 253 548**
**A2**

(12) # EUROPEAN PATENT APPLICATION

(21) Application number: **87305957.0**

(22) Date of filing: **06.07.87**

(51) Int. Cl.⁴: **G 01 N 33/72,** G 01 N 33/52, C 12 Q 1/28

(30) Priority: **15.07.86 IL 79417**
**10.06.87 IL 82840**

(43) Date of publication of application: **20.01.88**
**Bulletin 88/3**

(84) Designated Contracting States: **AT BE CH DE ES FR GB IT LI NL SE**

(71) Applicant: **SAVYON DIAGNOSTICS LTD., 1 Jabotinsky Street, Ramat-Gan (IL)**

(72) Inventor: **Ben-Michael, Abraham, 8 Rehavet Ilan Street, Ramat-Ilan Givat-Shmuel (IL)**

(74) Representative: **West, Alan Harry et al, R.G.C. Jenkins & Co. 26 Caxton Street, London SW1H 0RJ (GB)**

(54) Method for the determination of occult blood and chemical compositions therefor.

(57) A method is disclosed for the determination of pseudo-peroxidases, especially for the determination of occult blood in specimens of human material, which employs stable liquid chemical compositions containing chromogenic materials and a peroxide. Stable liquid chemical compositions for carrying out the method are also described.

1

## METHOD FOR THE DETERMINATION OF OCCULT BLOOD
## AND CHEMICAL COMPOSITIONS THEREFOR

The present invention relates to liquid stable chemical compositions for the determination of pseudo-peroxidases, especially haemoglobin, more particularly for the determination of occult blood in specimens of human material, and to a method for determining the presence of occult blood in such specimens, by using the said compositions.

The art has been searching for a long time for a way to improve the stability of test compositions for the determination of occult blood in specimens of human material. Such attempts, however, have led to the preparation of solid reagents, such as impregnated carrier matrices in the form of test sticks or strips. One of such compositions is described in U.S. Patent 4,556,640 in which different solutions are prepared, containing an organic hydroperoxide, a benzidine indicator and an aniline as stabilizing agent. Other materials are added to the solutions, such as surfactants, to facilitate dispersion and impregnation of the reactants in the carrier matrix. The compositions prepared according to this patent are stable in the liquid state for short periods of time, but present good stability when dried and kept in the form of solid reagent strips. No successful attempt has been done in the art to obtain reagents of this kind, which are stable in the liquid state, and which can be stored and used in the liquid state for carrying out test determinations of occult blood.

The peroxide employed in the art is an organic peroxide, typically cumene hydroperoxide, for occult blood testing, because of its specificity to haemoglobin. Detection of occult blood is carried out, in practice, by bringing the specimen containing occult blood into contact with the test composition. Haemoglobin, which acts as a pseudoperoxidase, reacts - if present in the specimen - with cumene hydroperoxide. This reaction causes the indicator to change its color. In this way, presence of haemoglobin is detected by the change of color of the reagent, which is normally an impregnated strip or stick.

The use of solid reagents presents several drawbacks, notably, the preparation of such solid reagents is relatively long and complex, since it requires separate application of the liquid reagents and intermediate drying thereof. In contrast, the preparation of liquid reagents is simple, quick, and the change in color of such reagents can be quantitatively determined, through spectrophotometric readings or the like. It is therefore clear that providing chemical reagents that can be both stored for long periods of time and then used in the liquid state, for the determination of occult blood in specimens of human material, is highly desirable and advantageous.

3.

It has now been found, and this is an object of the present invention, that it is possible to provide a method for the laboratory testing of occult blood employing liquid reagents that can be stored for long periods of time, such reagents being kept always in the liquid state.

It has further been found, and this is another object of the invention, that it is possible to carry out the determination of occult blood in specimens of human material, by using the compositions of the invention, in the liquid state, without the need for solid or impregnated reagent matrixes.

It has also been found, and this is still another object of the invention, that the use of hydrogen peroxide in the method and compositions of the invention, instead of an organic hydroperoxide, permits to avoid false negative responses which are common when organic hydroperoxides are employed, viz., negative results in tests which should have indicated the presence of occult blood, but failed to do so.

The stable liquid compositions according to the invention are characterized by a critical selection of the components, and contain:
a) a water-miscible aprotic solvent selected from among dimethylsulphoxide, dimethylformamide or dimethylacetamide;
b) an aqueous solvent medium;
c) an effective amount of chelating agents, selected from among tri sodium citrate, sodium pyrophosphate, acetanilide, 8-hydroxy-quinoline or a hemi-sulphate salt thereof, or a mixture of two or more of said chelating agents;

d) a stabilizing compound selected from among Guaiacol sulfonic acid, vanillin, vanillic acid, N-benzylidene methylamine, N-benzylidenebenzylamine, 10-benzylidene-9-anthrone, 2-(2-hydroxyphenyl)-benzoxazole, 1-hydroxybenzotriazole, 2-hydroxy-benzophenone and 2,4,4'-trihydroxy- benzophenone, flavianic acid, 2-pyrrolidinone, o-toluamide, p-toluamide, m-toluamide, p-nitrobenzamide, 1-methyl-2-pyrrolidinone, salicylanilide, 2,4,6-collidine, 2-quinoxalinol, 4-methylpyrimidine, benzamide, 4'-nitroacetanilide, ethyl nicotinate, 2-amino-4,6-dimethylpyrimidine, 2-(aminomethyl)pyridine, 4-pyridine carboxylic acid, 2,6-lutidin, 2-amino-6-methylpyridine, or a mixture of two or more of such stabilizing compounds;

e) a peroxide selected from the group consisting of organic hydroperoxides, preferably Cumene Hydroperoxide, and hydrogen peroxide and its analogues; and

f) an indicator selected from tetramethylbenzidine and its derivatives and tetramethyl- p-phenylendiamine.

Preferably, the compositions contain from about 100 to about 10,000 ppm of the stabilizing compound and, still preferably, from about 100 to about 10,000 ppm of chelating agents. Preferred aqueous solvent mediums are buffer solutions.

The compositions according to the invention can be strongly basic or strongly acidic, depending on the conditions in which favourable conditions are obtained for the dissolution and the color development of the indicator used. A strongly acidic composition, for example, contains, for each 1000 ml:

a) about 300 ml dimethylsulphoxide or dimethylformamide or dimethyl acetamide, preferably about 250 to about 350 ml;

b) about 700 ml distilled water, preferably about 650 to about 750 ml;

c) about 100 gr citric acid or acetic acid, preferably about 50 to about 150 gr;

d) about 10 gr tri sodium citrate, about 10 gr sodium pyrophosphate, about 10 gr acetanilide, preferably about 5 to about 15 gr of each, and about 5 gr 8-hydroxyquinoline hemi-sulphate salt, preferably 0 to about 10 gr;

e) about 10 gr guaiacol sulphonic acid or vanillin, preferably about 5 to about 15 gr;

f) about 1 gr tetramethylbenzidine, preferably about 0.5 to about 2 gr; and

g) about 1000 microliters hydrogen peroxide or Cumene hydroperoxide or a derivative thereof, preferably about 500 to about 1500 microliters.

The pH of such compositions is preferably between 3 and 4.

6

Strongly basic compositions are, for instance, those which contain, for each 1000 ml:

a) about 300 ml dimethylsulphoxide or dimethylformamide or dimethyl acetamide, preferably about 250 to about 350 ml;

b) about 700 ml distilled water, preferably about 650 to about 750 ml;

c) about 100 gr sodium carbonate, preferably about 50 to about 150 gr;

d) about 10 gr tri sodium citrate, about 10 gr sodium pyrophosphate and about 10 gr acetanilide, preferably about 5 to about 15 gr of each;

e) about 10 gr guaiacol sulphonic acid, preferably about 5 to about 15 gr, or about 5 gr vanillin or vanillic acid, preferably about 0 to about 15 gr;

f) about 1 gr tetramethyl-p-phenylendiamine, alone or in admixture with a color enhancing material selected from among 4-aminoantipyrine and/or 2,2'-azino-di-(3-ethylbezothiazoline sulfonic acid) and/or N-1-naphthyl ethylendiamine, preferably about 0.5 to about 2 gr of mixture; and

g) about 1000 microliters hydrogen peroxide or Cumene hydroperoxide, preferably about 500 to about 1500 microliters.

The pH of such compositions is preferably of between 10 and 11. Preferred compositions of this kind may further contain up to 10 gr N-benzylidene methylamine, N-benzylidene benzylamine, 10-benzylidene-9-anthrone, 2-(2-hydroxyphenyl)- benzoxazole, 1-hydroxybenzotriazole, 2-hydroxy-benzophenone or 2,4,4'-trihydroxy-benzophenone, or a mixture of two or more of said compounds.

The stable liquid compositions of the invention are preferably prepared by stages. Firstly, all reagents soluble in the aqueous medium are dissolved in such medium, and all reagents soluble in the aprotic solvent are dissolved in the aprotic solvent. Secondly, the aqueous medium is slowly added to the aprotic solvent, under stirring, care being taken to avoid too strong heat effects and temperature increases with solvents having a relatively high heat of solution in water, such as DMSO. Finally, the peroxide is added to the solution. Each addition step requires the application of stirring for periods of time appropriate for obtaining homogeneous dissolution of the various components, as exemplified hereinafter.

The method for determining the presence of occult blood in specimens of human material, according to the invention, is characterized in that the specimen to be tested is brought into contact with a liquid composition according to the invention, and it is checked whether color evolves.

The use of N,N,N',N'-tetramethyl-p-penylenediamine·2HCl (referred to in the specification as tetramethyl-p-phenylenediamine, for the sake of brevity) as a chromogen or indicator in colometric determinations is also novel and, as such, also forms part of the present invention.

Because of the very high sensitivity of compositions according to the invention, containing hydrogen peroxide, an occult blood determination carried out with such compositions may give false positive responses, but virtually no false negative responses. Because of the nature of the occult blood test, which is directed to obtaining a primary screening of patients which are potentially affected with bleeding of the digestive tracts, a false negative response may be dangerous. In practice, when a patient has been found positive in the occult blood test, more direct determinations of the cause of bleeding are done, such as gastroscopy, to locate the source of such blood. Since a common cause of bleeding is cancer, a false negative response to the occult blood test may cause a delay in the diagnosis and treatment of such a patient, which may seriously endanger him. Patients found to be positive to the occult blood test, on the other hand, are more thoroughly and closely examined, so that no danger results from a false positive response to this test. It is therefore clear that, from a clinical viewpoint, a larger number of false positive responses is preferred to the possibility of false negative ones.

The compositions of the invention, containing hydrogen peroxide instead of an organic hydroperoxide, almost entirely eliminate the danger of false negative results. Hydrogen peroxide, as herein defined, comprises hydrogen peroxide analogues such as urea hydrogen peroxide.

The above and other objects and advantages of the invention will be better understood through the following illustrative and non-limitative examples.

Example 1

Preparation of 1000 ml stable liquid reagent containing tetramethyl benzidine (TMB) as the chromogen.

Preparation of solution A. Into 300 ml dimethylsulphoxide (DMSO) there were dissolved 5.0 gr 8-hydroxyquinoline hemi-sulfate salt, 1.0 gr TMB, 10 gr guaiacol sulphonic acid and 10 gr acetanilide.

Preparation of solution B. Into 700 ml of distilled water there were dissolved 10 gr tri sodium citrate, 100 gr citric acid and 10 gr sodium pyrophosphate.

Preparation of final solution. Solution B was slowly poured under stirring into solution A, care being taken to avoid sharp temperature increases. The resulting solution was kept under stirring for 12 hours, to obtain full mixing. Thereafter, the solution was titrated to pH 3.5, with a 1N solution of HCl. Finally, 1000 microliters cumene hydroperoxide were added under stirring, and the solution was further stirred for 3 hours.

The resulting solution was stable and active for one month at 37°C, for six months at 25°C and for one year at 4°C.

Example 2

Example 1 was repeated, but using acetic acid instead of citric acid. The results obtained were as in Example 1.

Example 3

Example 1 was repeated, by replacing guaiacol sulphonic acid with vanillin. The results obtained were as in Example 1 above.

Example 4

Example 1 was repeated twice, using first dimethylformamide (DMF) and then dimethylacetamide (DMAC) instead of DMSO. The results obtained in each case were as in Example 1.

Example 5

Preparation of 1000 ml stable liquid reagent containing tetramethyl p-phenylenediamine (TMpPD) as the chromogen.

Preparation of solution A. Into 300 ml dimethylsulphoxide (DMSO) there were dissolved 1.0 gr TMpPD, 10 gr guaiacol sulphonic acid and 10 gr acetanilide.

Preparation of solution B. Into 700 ml distilled water there were dissolved 100 gr sodium carbonate, 10 gr sodium pyrophosphate and 10 gr tri-sodium citrate.

Preparation of final solution. Solution B was slowly added to solution A, under stirring, care being taken to avoid strong temperature increases. Stirring was continued for 12 hours and the solution was then brought to pH 11 by titrating it with a 10N NaOH solution. 1000 Microliters cumene hydroperoxide were added under stirring, and stirring was continued for 3 additional hours. The solution so obtained was stable for 3 weeks at 37°C, for 5 months at 25°C and for 10 months at 4°C.

Example 6

Example 5 was repeated twice, using first 5.0 gr vanillin and then 5.0 gr vanillic acid, instead of guaiacol sulphonic acid. The results obtained in each case were as in Example 5.

Example 7

Example 5 was repeated, but using triethanolamine instead of sodium carbonate. The results were as in Example 5.

Example 8

Example 5 was repeated, using DMAC instead of DMSO. The results obtained were as in Example 5.

Example 9

Preparation of a composition containing TMpPD, 2,2'-azino-di-(3-ethylbenzothiazoline sulfonic acid) (ABTS) and 4-aminoantipyrine (AAP) as the indicators.

Preparation of solution A. Into 300 ml dimethylsulphoxide (DMSO) there were dissolved 1.0 gr TMpPD, 1 gr ABTS and 1 gr AAP, and further 1 gr N-benzylidene methylamine, 1 gr N-benzylidenebenzylamine, 1 gr 10-benzylidene-9-anthrone and 10 gr acetanilide.

Preparation of solution B. Into 700 ml distilled water there were dissolved 100 gr di-sodium tetraborate-10-hydrate, 10 gr sodium pyrophosphate and 10 gr tri-sodium citrate.

Preparation of final solution. Solution B was slowly added to solution A, under stirring, care being taken to avoid strong temperature rise. Stirring was continued for 12 hours and the solution was then brought to pH 10 by titrating it with a 10N NaOH solution. 1000 Microliters cumene hydroperoxide were added under stirring, and stirring was continued for 3 additional hours. The solution so obtained was stable for 3 weeks at 37°C, for 5 months at 25°C and for 10 months at 4°C.

## Example 10

Example 9 was repeated, using sodium carbonate-10-hydrate instead of di-sodium tetraborate-10-hydrate. The results obtained were as in Example 9.

## Stability Test

In all above examples, stability of a given composition is determined by its activity towards haemoglobin at a given time, compared with the activity of a freshly prepared identical composition. A given composition is deemed to have lost stability when it either becomes inactive towards haemoglobin, or becomes turbid.

## Example 11
### Occult Blood in feces.
Two specimens of feces, one of which is suspected of containing occult blood, according to independent tests, were placed in thin layer, by means of a spatula, on a strong white absorbent paper. On each specimen there were dripped 0.5-1 ml of the solution of Example 1. Color evolved on the specimen suspected of containing occult blood and on the absorbent paper around it, pointing to the presence of haemoglobin in the specimen. No color evolved on or around the specimen found to be free from haemoglobin by independent testing. The test was repeated with the reagents of Examples 2 through 10, leading to comparable results. The test was found to give significant result at concentrations as low as 0.5 ml of blood for 100 gr of feces.

Example 12

Occult Blood in urine

A sample of 10-20 ml urine, known to contain occult blood from independent determinations, were placed into a test tube. To the test tube there were added 100-250μl of the composition of Example 1. Color evolved immediately. No color evolved when the same composition was added to a sample known to be free from occult blood. Testing with the compositions of Examples 2 through 4 gave comparable results.

Example 13

Preparation of 1000 ml stable liquid reagent containing tetramethyl benzidine (TMB) as the chromogen, and $H_2O_2$.

Preparation of solution A. Into 300 ml dimethylsulphoxide (DMSO) there were dissolved 5.0 gr 8-hydroxyquinoline hemi-sulfate salt, 1.0 gr TMB, 10 gr guaiacol sulphonic acid and 10 gr acetanilide.

Preparation of solution B. Into 700 ml of distilled water there were dissolved 10 gr tri sodium citrate, 100 gr citric acid and 10 gr sodium pyrophosphate.

Preparation of final solution. Solution B was slowly poured under stirring into solution A, care being taken to avoid sharp temperature increases. The resulting solution was kept under stirring for 12 hours, to obtain full mixing. Thereafter, the solution was titrated to pH 3.5, with a 1N solution of HCl. Finally, 500 microliters hydrogen peroxide were added under stirring, and the solution was further stirred for 3 hours.

The resulting solution was stable and active for one month at 37°C, for six months at 25°C and for one year at 4°C.

Example 14

Example 13 was repeated, but using 1 g of urea hydrogen peroxide. The results obtained were as in Example 13.

## Example 15

## Screening of Occult Blood in feces.

200 Patients suspected of diseases involving occult blood in feces were tested with two compositions. The first composition was the composition of Example 13, and the second was the composition of Example 1. Each specimen of feces was placed in thin layer, by means of a spatula, on a strong white absorbent paper. On each specimen there were dripped 2-3 ml of the test solution. If color evolved on the specimen and on the absorbent paper around it, the test was marked "positive".

The results of these tests are detailed in Table I below.

### Table I

| Patient | Cumene | $H_2O_2$ | Idependent |
|---------|--------|----------|------------|
| A | + | + | + |
| B | − | + | − |
| C | − | + | + |
| D | + | + | + |
| E | − | + | − |
| F | − | + | − |
| G | − | + | + |
| H | − | + | − |
| I | + | + | + |
| J | + | + | + |

In Table I, patients found positive in a given test are marked "+", while those found negative are marked "-" under the appropriate test. The column "Independent" refers to an independent test of the patient, such as gastroscopy, carried out to verify the results of the occult blood tests. As it is seen from Table I, patients, A, D, I and J were positive both to the Cumene and the $H_2O_2$ test, and were found to bleed in the independent test. Patients B, E, F and H were negative to the Cumene test, positive in the $H_2O_2$ test, and were found to be healthy according to the independent test (false positive). However, patients C and G, which were found to bleed in the independent test, were positive to the $H_2O_2$ test, but were negative to the Cumene test (false negative). Therefore, patients C and G, if tested only with compositions containing Cumene hydroperoxide, which is employed also in compositions according to the known art, would have been considered healthy persons and would have not been treated further. This example illustrates the importance of avoiding false negative responses, even at the cost of obtaining false positive results.

The above examples have been given for the purpose of illustration, and are not intended to be limitative. Many different modifications of the invention are possible. For instance, different comparable solvents can be employed, or different chelating agents can be added in quantities that provide effective chelating activity, and modifications of quantities, proportions or additives can be carried out, without exceeding the scope of the invention.

CLAIMS

1. A stable liquid composition for use in determining the presence of occult blood in specimens of human material, comprising

(a) a water-miscible aprotic solvent selected from di-methylsulphoxide, dimethylformamide and dimethyl-acetamide;

(b) an aqueous solvent medium;

(c) an effective amount of a chelating agent selected from tri-sodium citrate, sodium pyrophosphate, acetanilide, 8-hydroxy-quinoline and the hemi-sulphate salt thereof, and mixtures of any two or more such chelating agents;

(d) a stabilizing compound selected from guaiacol sulfonic acid, vanillin, vanillic acid, N-benzylidene-methylamine, N-benzylidenebenzylamine, 10-benzylidene-9-anthrone, 2-(2-hydroxyphenyl)-benzoxazole, 1-hydroxybenzotriazole, 2-hydroxy-benzophenone, 2,4,4'-trihydroxy-benzophenone, flavianic acid, 2-pyrrolidinone, o-toluamide, p-toluamide, m-toluamide, p-nitrobenzamide, 1-methyl-2-pyrrolidinone, salicylanilide, 2,4,6-collidine, 2-quinoxalinol, 4-methylpyrimidine, benzamide, 4'-nitroacetanilide, ethyl nicotinate, 2-amino-4,6-dimethylpyrimidine, 2-(aminomethyl)pyridine, 4-pyridine carboxylic acid, 2,6-
lutidine, 2-amino-6-methylpyridine, and mixtures of
any two or more such stabilizing compounds;

(e) an organic peroxide, preferably cumene hydroperoxide
or hydrogen peroxide or an analogue thereof; and

(f) an indicator selected from tetramethylbenzidine and
its derivatives and tetramethyl-p-phenylenediamine.


2. A composition according to claim 1, characterised
in that it contains from 100 to 10,000 ppmw of the
stabilizing compound.


3. A composition according to claim 1 or claim 2,
characterised in that it contains from 100 to 10,000 ppmw
of chelating agent.


4. A composition according to any one of claims 1 to
3, characterized in that the aqueous solvent medium is a
buffer solution.


5. A composition according to any one of claims 1 to
4, characterized in that it contains, per 1000 ml:

(a) from 250 to 350 ml of dimethylsulphoxide or dimethylformamide or dimethyl acetamide;

(b)  from 650 to 750 ml of distilled water;

(c)  from 50 to 150 g of citric acid or acetic acid;

(d)  from 5 to 15 g each of tri-sodium citrate, sodium pyrophosphate and acetanilide, and optionally up to 10 g or 8-hydroxyquinoline hemi-sulphate;

(e)  from 5 to 15 g of guaiacol sulphonic acid or vanillin;

(f)  from 0.5 to 2.0 g of tetramethylbenzidine; and

(g)  from 500 to 1500 $\mu$l of hydrogen peroxide or an analogue thereof, or of cumene hydroperoxide or a derivative thereof.


6.    A composition according to claim 5, having a pH from 3 to 4.


7.    A composition according to any one of claims 1 to 4, characterized in that it contains, per 1000 ml:

(a)  from 250 to 350 ml of dimethylsulphoxide or dimethylformamide or dimethyl acetamide;

(b)  from 650 to 750 ml of distilled water;

(c)  from 50 to 150 g of sodium carbonate;

(d)  from 5 to 15 g each of tri-sodium citrate, sodium pyrophosphate and acetanilide;

(e)  from 5 to 15 g of guaiacol sulphonic acid or, optionally, up to 15 g of vanillin or vanillic acid;

(f)  from 0.5 to 2 g of tetramethyl-p-phenylenediamine alone or in admixture with 4-aminoantipyrine and/or 2,2'-azino-di-(3-ethylbenzothiazoline sulfonic acid) and/or N-1-naphthyl ethylenediamine; and

(g) from 500 to 1500 µl of hydrogen peroxide or an analogue thereof, or of cumene hydroperoxide or a derivative thereof.

8. A composition according to claim 7, having a pH from 10 to 11.

9. A composition according to claim 7 or claim 8, characterized in that it further contains up to 10 g N-benzylidenemethylamine, N-benzylidenebenzylamine, 10-benzylidene-9-anthrone, 2-(2-hydroxyphenyl)-benzoxazole, 1-hydroxybenzotriazole, 2-hydroxy-benzophenone, 2,4,4'-trihydroxy-benzophenone or a mixture of any two or more such compounds.

10. A method for determining the presence of occult blood in specimens of human material, characterised in that the specimen to be tested is brought into contact with a liquid composition according to any one of claims 1 to 9, and the composition is monitored for the development of colour.

- 23 -

11.   A novel indicator for carrying out chromogenic determinations of occult blood, characterised in that it is tetramethyl-p-phenylenediamine.